# EUROPEAN PATENT APPLICATION

(11) **EP 3 943 013 A1**
(43) Date of publication of application: **26.01.2022**
(21) Application number: 20187645.5
(22) Date of filing: 24.07.2020
(51) Int. Cl.: A61B 8/12, A61B 5/00, A61B 5/021, A61B 5/0215, A61B 8/00, B06B 1/02, G01L 9/00, G01N 29/24, H01L 29/84, G01S 15/89

(54) **DEVICE AND SYSTEM DEVICE FOR ULTRASOUND IMAGING AND PRESSURE SENSING**

(71) Applicant: Koninklijke Philips N.V., 5656 AG Eindhoven (NL)
(72) Inventor: HAARTSEN, Jaap Roger, 5656 AE Eindhoven (NL); VAN DER HORST, Arjen, 5656 AE Eindhoven (NL); NGUYEN, Thanh Trung, 5656 AE Eindhoven (NL); MOUNAIM, Amine, 5656 AE Eindhoven (NL)
(74) Representative: Philips Intellectual Property & Standards

(57) **Abstract**

A device has an ultrasound head at a distal portion of a shaft, such as a catheter or guidewire. The ultrasound head comprises one or more cMUT elements. A controller comprises an ultrasound transmit circuit and an ultrasound receive circuit, for imaging or Doppler flow imaging. It also has a measuring circuit for measuring a pressure-dependent electrical characteristic of one or more of the cMUT devices to derive body liquid pressure values. Thus, the same device uses the same integrated circuit for both ultrasound imaging and pressure sensing.

## Description

### FIELD OF THE INVENTION

This invention relates to devices which incorporate ultrasound imaging at the tip of a shaft, such as a catheter.

### BACKGROUND OF THE INVENTION

Assessing the functional significance of cardiovascular and peripheral vascular disease by intravascular pressure and /or flow measurement has been shown beneficial to guide treatment of atherosclerotic disease. For this purpose, the use of catheter-based imaging as well as hemodynamic sensing are common clinical practices to assess the disease state of the vasculature.

Guide-wires with pressure and flow sensors close to the tip have been developed to perform these kind of measurements. Specifically for coronary arteries, indices have been developed to guide the cardiologist in the decision whether or not a lesion should be treated. Indices based on pressure (e.g. fractional flow reserve (FFR), instantaneous wave free ratio (iFR)) and flow (e.g. coronary flow reserve (CFR)) or a combination of pressure and flow (hyperemic stenosis resistance (HSR)) have been shown to be superior in guiding treatment decisions compared to angiographic assessment alone.

Furthermore, recently pulse wave velocity measurements, derived from simultaneous pressure and flow, have shown to be relevant in guiding treatment decisions in coronary and renal denervation treatment.

Intravascular ultrasound (IVUS) imaging catheters, such as inter-cardiac ultrasound (ICE) catheters, are well known. They typically incorporate piezoelectric or single crystal ultrasound elements at the catheter tip, for performing localized imaging. These catheters enable imaging of the inside of a blood vessel.

There are two types of IVUS catheters on the market: phase array catheters and rotational catheters. IVUS catheters provide detailed and accurate measurements of lumen and vessel size, plaque area and volume, and the location of key anatomical landmarks. They allow a physician to evaluate the size of a lesion and based on the original lumen size to select the treatment device (i.e. a stent). After treatment, IVUS can be used to evaluate treatment success, e.g. by determining the deployment of a stent.

Capacitive micro-machined ultrasound (cMUT) transducers are being developed both for the purpose of pressure sensing and for use in IVUS catheters. cMUT devices are becoming increasingly popular because they can offer excellent bandwidth and acoustic impedance characteristics, which makes them the preferable over e.g. piezoelectric transducers. Vibration of the cMUT membrane can be triggered by applying pressure (for example using ultrasound) or can be induced electrically. Additionally, cMUT devices have been shown to be beneficial in Doppler ultrasound (US) on a catheter/guide-wire for flow velocity sensing. Another application area for a cMUT device is to receive US signals to localize its position in an external ultrasound field.

In such a cMUT transducer there are one or more drums. Such a drum comprises a deflectable membrane suspended over a vacuum cavity. There are two electrodes, one fixed electrode located in the bottom of the cavity and one electrode in the membrane. By applying an AC signal at an ultrasonic frequency across the electrodes, the membrane will vibrate and the cMUT drum will transmit ultrasonic waves. If ultrasonic waves are applied on the membrane of a biased cMUT cell, it will generate an alternating signal as the capacitance of the cMUT drum is varied. In this way, it works as a receiver of ultrasonic waves.

If the pressure difference across the membrane is changed, the electrical capacitance will change and hence the cMUT drum works as a pressure transducer.

cMUT ultrasound and pressure transducers can be operated in two different modes, i.e. conventional (non-collapse) and collapse mode. In collapse mode, the membrane is partially in contact with the bottom of the cavity at all times. This can be achieved either by design (whereby the ambient pressure is sufficient to push the membrane into 'collapse') or by applying a sufficiently high bias voltage to attract the membrane towards the bottom of the cavity.

cMUT array devices require local integrated circuitry (in particular ASICs) to enable operation of the cMUT elements. For example, high voltage pulses of a pulser circuit and a high voltage DC bias are generated by a probe circuit, which is typically an application specific integrated circuit (ASIC) which is located within the ultrasound probe, i.e. at the probe location. The ASIC is for example underneath the cMUT drums. The ASIC facilitates both transmission and reception modes of the device. In reception mode, changes in the membrane position cause changes in electrical capacitance, which can be registered

electronically. In transmission mode, applying an electrical signal causes vibration of the membrane. Besides the ASICs, several capacitors are also needed to enable a stable power supply to the ASIC and cMUT. These capacitors are typically rather large.

Currently, assessing both the pressure and imaging the blood vessels requires two separate devices. Being able to combine this in a single device would be beneficial from both a workflow and cost point of view. Furthermore, having a single transducer array to perform both functions would be advantageous from an integration/manufacturing point of view.

However, the combination of the different sensor functions is not simple to achieve.

### SUMMARY OF THE INVENTION

The invention is defined by the claims.

According to an aspect of the invention a device is provided, comprising: a shaft; a cMUT device including one or more cMUT elements at a distal portion of the shaft; an integrated circuit controller connected to the cMUT device, comprising:
an ultrasound transmit circuit and an ultrasound receive circuit configured for operation of one or more cMUT elements in ultrasound imaging mode and/or body liquid flow velocity measurement mode;
a measuring circuit configured for measuring a pressure-dependent electrical characteristic of one or more cMUT elements and deriving pressure measurement values of body liquid.

The ultrasound imaging is preferably of body anatomy including body structures of a patient and body liquids. An example of body liquid may be blood in the blood vessels or in organs such as heart.

In an embodiment of the device the same one or more cMUT elements of the cMUT device interface with the body liquid to provide simultaneous or interlaced pressure measurement of the body liquid, ultrasound imaging and/or body liquid flow velocity measurement values. It provides simplicity of the device, as the same sensor(s) or cMUT elements can be used at the same location at the distal portion or distal end of the device to measure multiple physiological parameters such as blood flow velocity and blood pressure, while being capable of also providing ultrasound information of the body anatomy at the same location. Other benefit is that the device can better cope with space constraints having less sensor elements and being able to provide multiple different measurements with the same sensor element(s). Accordingly, the device is not only suitable for introducer sheath or catheter, but also as guidewire, for example usable in coronary vessels or cerebral blood vessels.

In some embodiments a first sub-array of the cMUT device is used for ultrasound transmission and reception and a second sub-array of the cMUT device is used for measuring pressure values of body liquid.

A tracking system for tracking the location of the device may be used, which is operable based on ultrasound emitted or received by the cMUT device, and wherein a display can further be configured to displaying location information of the device, besides ultrasound images, flow velocity information and pressure information.

According to a further example in accordance with the invention, there is provided an ultrasound device comprising: a shaft; an ultrasound head at a distal end of the shaft comprising an array of cMUT devices; an integrated circuit controller comprising:
an ultrasound transmit circuit;
an ultrasound receive circuit;
a measuring circuit for measuring a pressure-dependent electrical characteristic of one or more of the cMUT devices.

This device enables ultrasound based measurements such as for ultrasound imaging or Doppler flow velocity measurement, as well as pressure sensing. The use of a shared integrated circuit and the same array of devices for both sensing modalities avoids complicated wiring to multiple sensor types.

The controller for example comprises an ASIC monolithically integrated with the array of cMUT devices. This provides a low cost fully integrated solution.

The pressure-dependent electrical characteristic in one example comprises a capacitance and the measuring circuit comprises:
an oscillator circuit which incorporates the cMUT device; and
an output circuit for providing an output signal which depends on the oscillator frequency.

The oscillator circuit may have a single oscillator for all of the cMUT cells (or one oscillator circuit per semiconductor die) and the cells are connected electrically in parallel. The cMUT devices contribute an electrical capacitance to the oscillator circuit and this thereby alters the oscillation frequency. A signal at the oscillation frequency is the output signal from the measuring circuit.

Each cMUT device may be part of a transducer circuit which comprises a bias terminal for applying a bias voltage to the cMUT device and a stimulus terminal for applying a drive signal to the cMUT device, and wherein the measuring circuit comprises a phase locked loop circuit for regulating a bias voltage provided to the bias terminal to achieve a fixed frequency.

The bias voltage required to achieve the fixed frequency of oscillation then becomes the output of the measuring circuit.

The phase locked loop circuit for example comprises a capacitance-to-frequency converter local to the cMUT device, and a loop filter and frequency reference source remote from the cMUT device.

In another example, the pressure-dependent electrical characteristic comprises a resonance frequency. The cMUT device is for example operated in a non-collapse mode during measurement of the resonance frequency.

The measuring circuit may then comprise a resonance detection circuit which comprises:
a feedback controlled oscillator circuit; or
a circuit for monitoring an impulse response; or
an impedance measurement circuit.

In one set of examples, the same cMUT devices are used for ultrasound transmission and reception as for measuring a pressure-dependent electrical characteristic. This enables a simple uniform array of cMUT devices.

In another set of examples, a first sub-array of the cMUT devices is used for ultrasound transmission and reception and a second sub-array of the cMUT device is used for measuring a pressure-dependent electrical characteristic. This enables the cMUT devices of each sub-array to be optimized to their particular function. It also enables true simultaneous monitoring.

The controller may be operable in an ultrasound imaging mode, in which the transmit circuit delivers pulses to the cMUT devices and the ultrasound receive circuit receives echo signals, and a pressure sensing mode. The device thus combines imaging with pressure sensing.

The imaging mode may comprise a sequence of frames, and the pressure sensing mode comprises measurements performed between frames of the imaging mode. This provides a sequential pressure sensing mode and imaging mode. If the pressure is recorded between the individual ultrasound firings, the pressure may be recorded when the ultrasound image is being reconstructed. The pressure sensing and imaging are thus effectively determined in the same time frame.

The controller may be operable in a Doppler flow velocity measurement mode (instead of or as well as the imaging mode), in which the transmit circuit delivers pulses to the cMUT devices and the ultrasound receive circuit receives echo signals, and a pressure sensing mode.

The device for example comprises a catheter.

The invention also provides an ultrasound system comprising: an ultrasound device defined above and a display system for displaying as a combined image two or more of: ultrasound images;
flow velocity information;
pressure information;
angiogram images.

The system may further comprise a tracking system for tracking the location of the ultrasound device based on ultrasound emitted or received by the ultrasound head in an external ultrasound field, and the display is further displaying location information for the ultrasound device.

The invention also provides a method of performing ultrasound monitoring and pressure sensing using an ultrasound device comprising a shaft and an ultrasound head at a distal end of the shaft comprising an array of cMUT devices, wherein the method comprises:
using an integrated circuit controller to control an ultrasound transmit circuit and an ultrasound receive circuit to perform ultrasound monitoring; and
using the integrated circuit controller to measuring a pressure-dependent electrical characteristic of one or more of the cMUT devices.

The ultrasound monitoring may comprise ultrasound imaging or Doppler flow velocity monitoring.

### BRIEF DESCRIPTION OF THE DRAWINGS

Examples of the invention will now be described in detail with reference to the accompanying drawings, in which:
Figure 1 shows a schematic drawing of an exemplary ultrasound imaging catheter;
Figure 2 schematically depicts a typical cMUT element of a system operable in a collapsed mode;
Figures 3 and 4 depict operating principles of such a cMUT element;
Figure 5 shows a first example of a device in accordance with the invention;
Figure 6 shows the relationship between capacitance (y-axis) and pressure (x-axis) for various bias voltages applied to a cMUT device;
Figure 7 shows a time line with alternate imaging frames and pressure sensing periods;
Figure 8 shows a time line with pressure sensing periods every N imaging frames;
Figure 9 shows an alternative way to implement the measuring circuit for measuring a pressure-dependent electrical characteristic;
Figure 10 shows the relation between the pressure (x-axis) and the bias voltage Vbias (y-axis) of a cMUT device;
Figure 11 shows the measured resonance frequency (y-axis) of a non-collapse operated cMUT array as a function of pressure (x-axis);
Figure 12 shows another way to implement the measuring circuit for measuring a pressure-dependent electrical characteristic; and
Figure 13 shows a device with a first sub-array of the cMUT devices for ultrasound and a second sub-array for pressure sensing.

### DETAILED DESCRIPTION OF THE EMBODIMENTS

The invention provides an ultrasound device with an ultrasound head at a distal potion or distal end of a shaft, such as a catheter or a guidewire. The ultrasound head has an array of cMUT element, which can be regarded as a cMUT device. A controller comprises an ultrasound transmit circuit and an ultrasound receive circuit, for imaging and/or Doppler flow imaging. It also has a measuring circuit for measuring a pressure-dependent electrical characteristic of one or more of the cMUT devices. Thus, the same device uses the same integrated circuit for both ultrasound imaging and pressure sensing.

The invention will be described with reference to ultrasound imaging at the tip of a catheter.

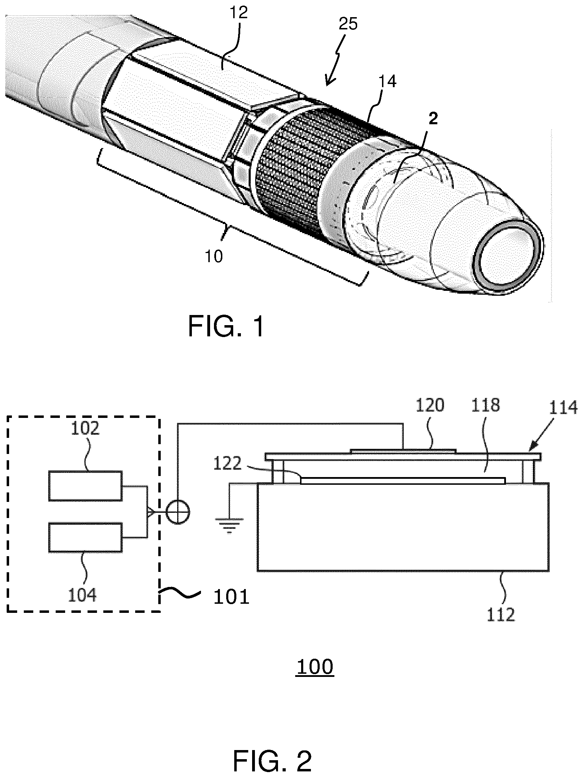

Figure 1 shows a schematic drawing of an example ultrasound imaging catheter.

The catheter has an outer diameter of approximately 1 mm. Internally, there is a hollow core 2, for example for guiding the catheter along a guide wire.

The catheter comprises an imaging core 10 having an array of integrated circuits 12. These are control ASICs mounted by flip-chip binding to supports, such as support dies.

These support dies are formed by the substrate of the flex-to-rigid (F2R) interconnect technology, as described below.

The integrated circuits have a length in the direction parallel to the length direction of the catheter and a width in the perpendicular direction. They are arranged around the catheter, with their width directions aligned around the catheter circumference.

The imaging core further comprises an array 14 of ultrasound transducers. The transducers basically comprise an acoustic generator and receiver in the form of a cMUT element. cMUT elements can be made by semiconductor processes and in particular the same processes used to produce the application specific integrated circuits (ASICs) needed by an ultrasound probe such as a CMOS process.

cMUT elements are tiny diaphragm-like devices with electrodes that convert the sound vibration of a received ultrasound signal into a modulated capacitance. Many such individual cMUT elements can be connected together and operated in unison as a single transducer device. For example, four to twenty four cMUT elements can be coupled together to function in unison as a single transducer element.

The manufacture of cMUT-based ultrasound systems is therefore more cost-effective compared to PZT-based systems. Moreover, due to the materials and dimensions used in such semiconductor processes, the cMUT elements exhibit much improved acoustic impedance matching to water and biological tissue, which obviates the need for (multiple) matching layers and yields an improved effective bandwidth.

cMUT elements in particular are able to function over a broad bandwidth, enable high resolution and high sensitivity imaging, and produce a large pressure output so that a large depth of field of acoustic signals can be received at ultrasonic frequencies.

Figure 2 shows a known design of cMUT element 100 (a so-called cell) for use in an ultrasound system and a known drive arrangement 101. The cMUT element 100 comprises a flexible membrane or diaphragm 114 suspended above a silicon substrate 112 with a gap or cavity 118 there between. A first electrode 122 is located on the floor of the drum on the upper surface of the substrate 112 in this example. A second electrode 120 is located on the diaphragm 114 and moves with the diaphragm. In the example shown, the two electrodes are circular.

A dielectric (not shown) is provided on the substrate 112 and underneath the top (second) electrode 120. The diaphragm may instead function as a dielectric layer.

Preferably, there are two dielectrics which may be equal in composition and thickness, but may be also asymmetric (different materials and thicknesses).

The membrane layer 114 is fixed relative to the top face of the substrate layer 112 and configured and dimensioned so as to define a spherical or cylindrical cavity 118 between the membrane layer 114 and the substrate layer 112.

Other realizations of the electrode 120 design can be considered, such as electrode 120 may be embedded in the membrane 114 or it may be deposited on the membrane 114 as an additional layer.

The first electrode may be directly exposed to the gap 118 or separated from the gap 118 by an electrically insulating layer or film to prevent a short-circuit between the second electrode 120 and the first electrode 122.

In Figure 2 the first electrode 122 is grounded by way of example. Other arrangements, e.g. a grounded second electrode 120 or both second electrode 120 and first electrode 122 floating are of course equally feasible.

The electrodes of the cMUT element 100 provide the capacitive plates of the device and the gap 118 is the main dielectric between the plates of the capacitor. When the diaphragm vibrates, the changing dimension of the dielectric gap between the plates provides a changing capacitance, which is sensed as the response of the cMUT element 100 to a received acoustic echo.

The spacing between the electrodes is controlled by applying a static voltage, e.g. a DC bias voltage, to the electrodes with a voltage supply 101. The voltage supply 101 may optionally comprise separate stages 102, 104 for providing the DC and AC or stimulus components respectively of the drive voltage of the cMUT elements 100, e.g. in transmission mode. The first stage 102 may be adapted to generate the static (DC) voltage component and the second stage 104 may be adapted to generate an alternating variable drive or stimulus voltage component having a set alternating frequency, which signal typically is the difference between the overall drive voltage and the aforementioned static component thereof.

The static or bias component of the applied drive voltage preferably meets or exceeds the threshold voltage for forcing the cMUT element 100 into its collapsed state. This has the advantage that the first stage 102 may include relatively large capacitors, e.g. smoothing capacitors, in order to generate a particularly low-noise static component of the overall voltage, which static component typically dominates the overall voltage such that the noise characteristics of the overall voltage signal will be dominated by the noise characteristics of this static component.

It is known that by applying a static voltage above a certain threshold, the cMUT element 100 is forced into a collapsed state in which the membrane 114 collapses onto the substrate 112. This threshold value may depend on the exact design of the cMUT element 100 and is defined as the DC bias voltage, known as the collapse voltage, at which the membrane 114 sticks to (contacts) the drum floor through the force due to the electric field between the electrodes. The amount (area) of contact between the membrane 114 and the substrate 112 is dependent on the applied bias voltage.

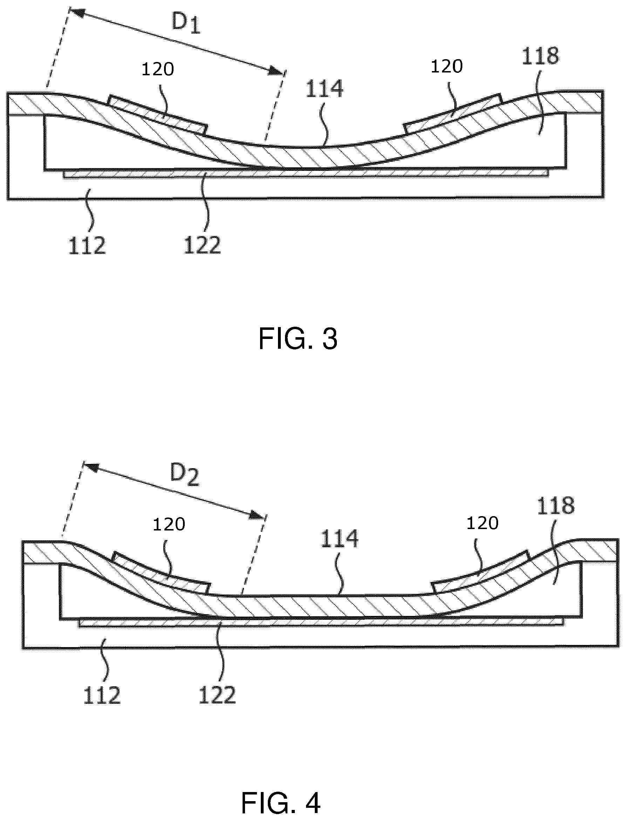

Increasing the contact area between the membrane 114 and the substrate 112 increases the resonant frequency of the membrane 114, as will be explained in more detail with the aid of Figure 3 and Figure 4.

The frequency response of a collapsed mode cMUT element 100 may be varied by adjusting the DC bias voltage applied to the cMUT electrodes after collapse. As a result, the resonant frequency of the cMUT element increases as a higher DC bias voltage is applied to the electrodes.

The principles behind this phenomenon are illustrated in Figures 3 and 4. The cross-sectional views of Figures 3 and 4 illustrate this one-dimensionally by the distances D1 and D2 between the outer support of the membrane 114 and the point where the membrane begins to touch the floor of the cavity 118 in each illustration. It can be seen that the distance D1 is a relatively long distance in Figure 3 when a relatively low bias voltage is applied, whereas the distance D2 in Figure 4 is a much shorter distance due to a higher bias voltage being applied. These distances can be compared to long and short strings which are held by the ends and then plucked in the one-dimensional drawing, however they are resembling to an inverted hollow conical frustum in a three-dimensional reality. The long, relaxed string will vibrate at a much lower frequency when plucked than will the shorter, tighter string. Analogously, the resonant frequency of the cMUT element in Figure 3 will be lower than the resonant frequency of the cMUT element in Figure 4, which is subject to the higher bias voltage.

Thus, a typical cMUT design comprises a flexible membrane or diaphragm, for example formed of silicon nitride, suspended above a silicon substrate with a gap or cavity between them. The gap is caused by removing a sacrificial layer during manufacturing. A first electrode is located on the floor of the drum on the upper surface of the substrate and a second electrode is located on the diaphragm and moves with the diaphragm, or it is integral part of the diaphragm. This same architecture may be used as a pressure sensor or as an ultrasound transmitter and receiver, the latter involves measuring the acoustical pressure of an ultrasound echo wave or even further deriving from the ultrasound information blood flow velocity.

The operation of cMUT elements will be well known to those skilled in the art.

The cMUT elements are formed on a flexible carrier so they can be wrapped around the periphery of the core 2 of the catheter, as shown in Figure 1. The integrated circuits can similarly be wrapped around the core of the catheter because despite being rigid, they are formed as separate fingers. The connections between the integrated circuits 12 and the array 14 is by means of flexible to rigid interconnects 25, preferably using the so-called F2R interconnection system.

F2R is a technology platform for the fabrication of miniature partially flexible sensor systems for minimally invasive medical instruments. Because of the flexibility, these sensor systems can be wrapped around cylindrical medical instruments such as catheters or guidewires. F2R is a post-processing technology whereby flexible interconnects, consisting of a metal routing layer sandwiched between two layers of flexible polyimide, are fabricated on a silicon wafer. This is followed by a separation step whereby individual elements are singulated by Deep Reactive Ion Etching (DRIE) and Reactive Ion Etching (RIE). The F2R fabrication is based on standard IC fabrication technology, which allows the devices to scale down so that all kind of sensing functionalities and electronics can be integrated into the tip of a catheter.

Currently, assessing both the pressure and imaging the blood vessels require two separate devices. The invention enables these two functions to be combined in a single device, giving benefits in workflow, manufacturing and cost points of view. In particular, the invention provides a cMUT ultrasound device comprising a shaft and an ultrasound head at a distal portion or distal end of the shaft. An integrated circuit controller has an ultrasound transmit circuit, an ultrasound receive circuit and a measuring circuit for measuring a pressure-dependent electrical characteristic of one or more of the cMUT devices. The device can thus send and receive ultrasound signals and measure pressure at the same location with a single transducer array.

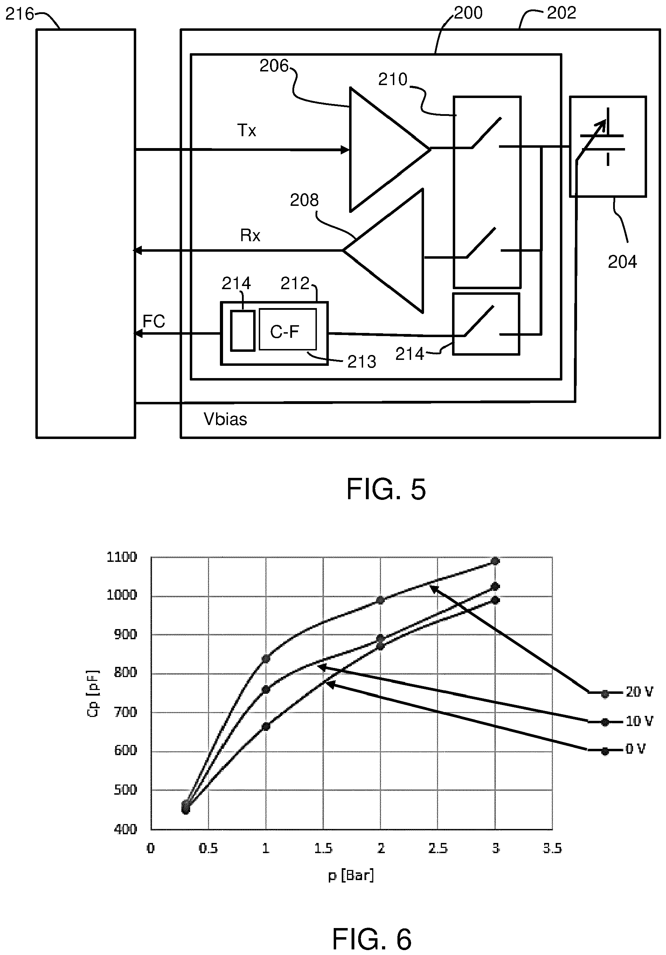

Figure 5 shows a first example of a device in accordance with the invention. The device comprises a controller 200, which is formed as part of an ASIC 202. An array of cMUT devices 204 is monolithically integrated with the ASIC 202. Each cMUT device may comprise a number of actual cMUT elements (i.e. cells) as mentioned above.

The controller 200 comprises an ultrasound transmit circuit 206 and an ultrasound receive circuit 208 for each cMUT device 204, for an imaging mode. A switch arrangement 210 for each cMUT device couples the receive and transmit circuits to the cMUT device 204. A patient interface module (PIM) 216 controls the timing of when to transmit and when to start receiving and hence controls the switch arrangements 210 for the different cMUT devices. The cMUT devices are connected to the transmit and receive circuits for an imaging mode, or else all cMUT devices are isolated from the transmit and receive circuits for a separate pressure sensing mode, also controlled by the PIM.

The controller further comprises a measuring circuit 212 for measuring a pressure-dependent electrical characteristic of the cMUT device. The measuring circuit 212 connects to the cMUT device through a second switch 214. Alternatively, of additionally a second switch 214 can be provided again for each cMUT device 204. However, a single measuring circuit 212 may be provided. Thus, the cMUT devices 204 may all be connected electrically in parallel for the pressure sensing mode. If there are multiple ASIC dies each with a respective sub-array of the cMUT devices, there may then be a measuring circuit per semiconductor die.

The pressure-dependent electrical characteristic of the cMUT device in this example comprises the capacitance of the device. To measure the capacitance, the measuring circuit 212 comprises an oscillator circuit 213, which combines with the cMUT device to define the oscillation characteristics, and an output circuit 214 for providing an output signal FC which depends on the oscillator frequency.

The measuring circuit 212 in this example comprises an oscillator 213 to measure the CMUT capacitance, which depends on the pressure being sensed. The oscillator is thus one example of a capacitance-to-frequency converter circuit.

The ASIC 202 connects to the patient interface module (PIM) 216, which provides the control commands for transmission, processes the received reflected echo signals, and receives the pressure measurement signal (output signal FC in this example). The module 216 also provides the bias voltage to the cMUT devices. The bias voltage is for example the same for all cMUT devices of the array.

Figure 6 shows the relationship between capacitance (y-axis) and pressure (x-axis) for various bias voltages applied to the cMUT devices.

During the ultrasound imaging mode, the PIM sends the request for a transmit event to the ASIC, and the ASIC then delivers the high voltage pulses to the cMUT devices. The ASIC receive the acoustic echo signal and sends it to the PIM. The switches of the switch arrangement 210 are closed and the switch 214 is open.

During the pressure sensor mode, the switches of the switch arrangement 210 are open, and the switch 214 is closed. The measuring circuit 212 of the ASIC converts the capacitance value of the cMUT into the output signal FC. The frequency of the signal FC and the capacitance of the cMUT are related.

When the pressure changes, the capacitance of the cMUT changes as shown in Figure 6, and hence the resonant oscillation frequency represented by the output signal FC changes accordingly.

The example of Figure 5 is based on converting the capacitance of the cMUT device (or more accurately the parallel combination of the cMUT devices of the ASIC) into a frequency. The capacitance may instead be converted into a signal having a duty cycle, voltage or current, which represents the capacitance. The cMUT capacitance may instead be converted into a digital signal.

An ultrasound image is formed of frames, where each frame consists of multiple fire and receive events. The pressure sensing can take place after each frame or after sequences of multiple frames. In this way the image generation and the pressure sensing are performed in real time.

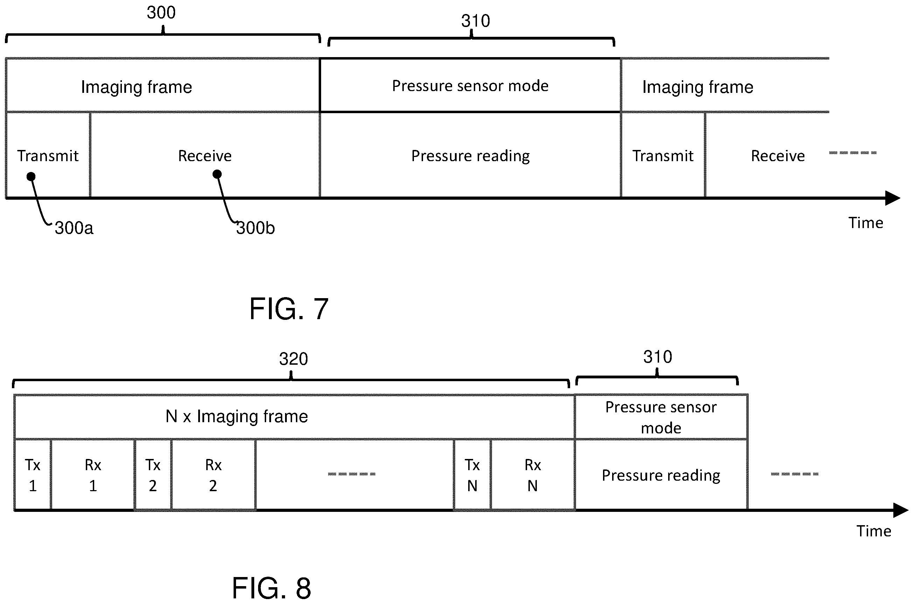

Figure 7 shows a time line with alternate imaging frames 300 and pressure sensing periods 310. Each imaging frame comprises a transmit period 300a and a receive period 300b. Typically, the frame duration is around 20µs and the pressure sensing has a duration of around 10µs.

This provides a sequential pressure sensing mode and imaging mode. The pressure may be sensed when the ultrasound image is being reconstructed. The pressure sensing and imaging are this effectively determined in the same time frame.

The pressure sensing does not need to be updated at such a fast rate (every 30µs in this example), so it is possible to reduce the time impact of the pressure sensing on the frame rate by reducing the rate at which pressure measurements are taken.

Figure 8 shows a time line with pressure sensing periods 310 every N imaging frames 320. Each imaging frame again comprises a transmit period and a receive period.

The value N can be of the order of 1000. With a resulting combined frame duration of 20ms, the pressure reading duration is negligible in comparison, but the pressure is updated at a sufficiently high rate (50Hz in this example). Even less frequent pressure measurement is of course possible.

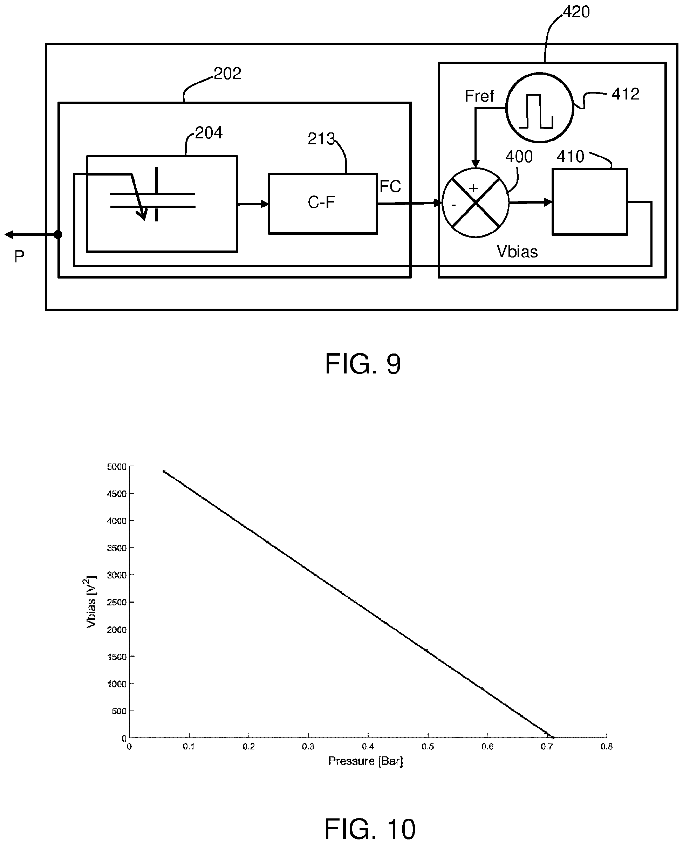

Figure 9 shows an alternative way to implement the measuring circuit for measuring a pressure-dependent electrical characteristic the cMUT devices. In this circuit, a closed loop feedback path is used to maintain the capacitance of the cMUT device constant by adjusting the bias voltage Vbias.

The circuit comprises the cMUT device 204 and an oscillator circuit 213 for converting capacitance to frequency, as explained above. The output frequency is compared by a comparator 400 with a fixed reference frequency Fref from a fixed frequency source 412. The circuit has a loop filter 410 to implement a phase locked loop (PLL) control system that compares the output frequency FC, which represents the cMUT capacitance, with the fixed reference frequency Fref, and tunes the bias voltage Vbias of the cMUT device, such that the output frequency is constant and equal to the reference frequency.

This means in practice that the cMUT device membrane is controlled to stay constantly in a certain position by adjusting the bias voltage in order to match the output frequency and the reference frequency. The bias voltage to keep the membrane in the fixed position is a measure of the pressure exerted on the membrane. The circuit unit 420 (loop filter, frequency reference, comparator) is for example at the PIM or it can be integrated in the proximal portion of the device that remains out of the body of the patient. There is one such circuit, connected to all the cMUT devices, and on the ASIC there is also one oscillator circuit 213 connected to the cMUT devices in parallel.

Thus, the capacitance-to-frequency converter of the phase locked loop circuit is local to the array of cMUT devices, whereas the loop filter and frequency reference source are remote from the array of cMUT devices.

Figure 10 shows the relation between the pressure (x-axis) and the bias voltage Vbias (y-axis) based on an analytical model of a cMUT device.

In another example, the pressure-dependent electrical characteristic comprises a resonance frequency of the cMUT device (rather than an oscillation frequency of an oscillator circuit which includes the cMUT device capacitance).

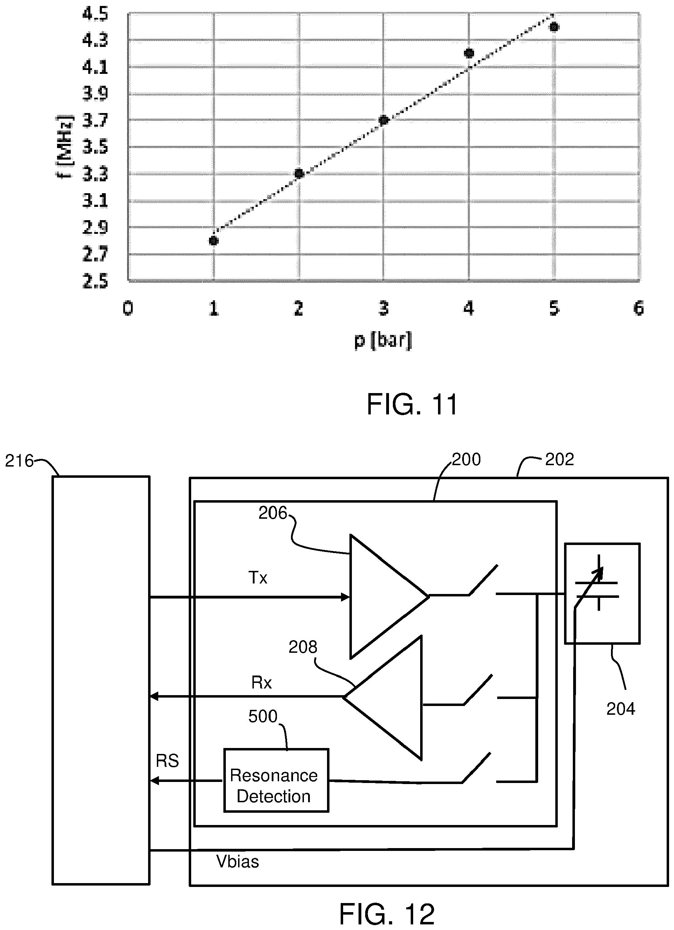

Figure 11 shows the measured resonance frequency (y-axis) of a non-collapse operated cMUT array as a function of pressure (x-axis). The cMUT devices can of course also be operated in collapse mode.

Figure 12 shows the circuit using resonance frequency measurement. It is equivalent to the circuit of Figure 5, again comprising a controller 200 which is formed as part of an ASIC 202. The array of cMUT devices 204 is monolithically integrated with the ASIC 202.

The capacitance to frequency converter 213 in Figure 5 is replaced by a resonance detection circuit 500 which generates a resonance signal RS. This circuit can also be monolithically integrated in the ASIC with the cMUT elements but it can instead be an external circuit in the PIM (or elsewhere).

Methods suitable for measuring a resonance frequency of the cMUTs are well known in the art.

A first possible approach is to drive the membrane (or multiple membranes) to the structural resonance by a feedback-controlled oscillator circuit configured to maintain the resonant membrane(s) at its most mechanically-efficient frequency or "maximum-Q" response point. A counter circuit may then be used to count the oscillator output over some defined time-averaging window. The frequency response of the resonant sensor is therefore a direct function of the number of time-averaged samples provided per second. Alternatively, the frequency of the resonant structure can be measured utilizing a period measurement system to provide a wider measurement bandwidth.

A second possible approach is to subject the membrane (or multiple membranes) to an impulse, e.g. a short voltage spike applied to the pressure variable capacitor. The membrane will vibrate at its resonance frequency. The frequency can then be measured by a counter or period measurement system as mentioned above.

A third possible approach is to perform impedance measurement, e.g. by applying an AC voltage to the biased cMUT device while sweeping the frequency around the resonance frequency. By measuring the corresponding current the impedance can be calculated as a function of frequency. The resonance frequency is then located where the phase of the impedance is maximal or where the magnitude of the impedance is maximal (hence the open circuit resonance).

In the examples above, the same cMUT devices are used for ultrasound transmission and reception as for measuring a pressure-dependent electrical characteristic.

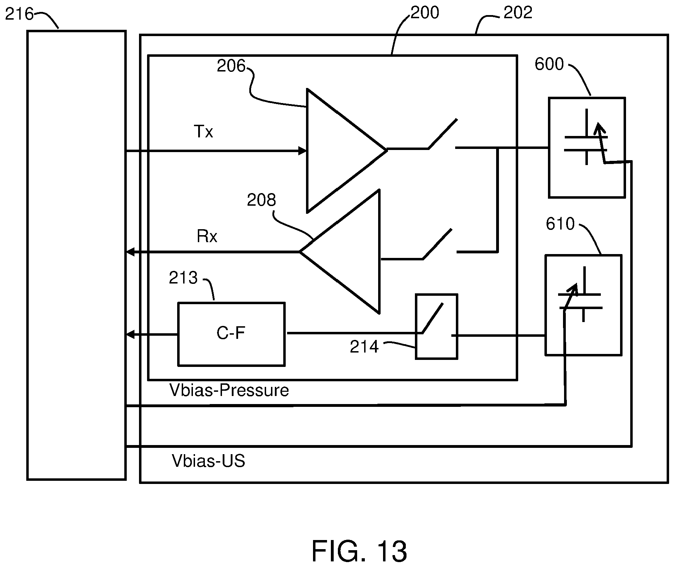

Figure 13 shows an alternative in which a first sub-array 600 of the cMUT devices is used for ultrasound transmission and reception and a second sub-array 610 of the cMUT device is used for measuring a pressure-dependent electrical characteristic.

The arrangement otherwise corresponds to Figure 5.

The cMUT devices of the two sub-arrays may have differently sized cMUT drums. Indeed, the bias voltage, dimensions, form factor and operating mode (collapse/conventional) of the cMUT elements of one sub-array can each then be optimized for ultrasound transduction while these parameters for the cMUT elements of the other sub-array are optimized for pressure sensing. The ASIC again comprises two circuits; the transmit and receive circuits for ultrasound imaging and the measuring circuit for pressure sensing. The circuits connect their respective sub-array of cMUT devices. The switch 214 is not in fact needed in this implementation.

The examples above are based on ultrasound imaging. However, the controller may instead or additionally be operable in a Doppler flow velocity measurement mode, in which the transmit circuit delivers pulses to the cMUT devices and the ultrasound receive circuit receives echo signals. When integrated on a catheter or guidewire, pressure and blood velocity are measured with the same array, and in addition the measurement locations for pressure and velocity are very close to each other. This enables vessel compliance and pulse wave velocity measurements with a single transducer array, at practically the same location in a vessel.

Another option is to track the position of the transducer array to enable mapping of the pressure and Doppler velocity in an ultrasound image. This approach is based on tracking an ultrasound signal emitted or received by the catheter in an ultrasound image, for example making use of the receive beamformer. Such external ultrasound tracking is for example described in US 10,130,330 B2.

The invention can be applied to all catheter-like ultrasound imaging products, such as needles, guidewires, using ultrasound or high intensity focused ultrasound (HIFU).

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A device comprising:
a shaft (14);
a cMUT device including one or more cMUT elements at a distal portion (10) of the shaft;
an integrated circuit controller (200) connected to the cMUT device, comprising:
an ultrasound transmit circuit (206) and an ultrasound receive circuit (208) configured for operation of one or more cMUT elements in ultrasound imaging mode and/or body liquid flow velocity measurement mode;
a measuring circuit (212) configured for measuring a pressure-dependent electrical characteristic of one or more cMUT elements and deriving pressure measurement values of body liquid.

2. The device of claim 1, wherein the same one or more cMUT elements of the cMUT device are configured to interfacing the body liquid to provide simultaneous or interlaced pressure measurement of the body liquid, ultrasound imaging and/or body liquid flow velocity measurement values.

3. The device of any one of claim 1 to 2, wherein the pressure-dependent electrical characteristic comprises a capacitance and the measuring circuit comprises:
an oscillator circuit (213) which incorporates the cMUT device; and
an output circuit for providing an output signal (FC) which depends on the oscillator frequency.

4. The device of claim 3, wherein each cMUT element of the cMUT device is part of a transducer circuit which comprises a bias terminal for applying a bias voltage to the cMUT device and a stimulus terminal for applying a drive signal to the cMUT device, and wherein the measuring circuit comprises a phase locked loop circuit (400, 410, 420) for regulating a bias voltage (Vbias) provided to the bias terminal to achieve a fixed frequency (Fref).

5. The device of claim 4, wherein the phase locked loop circuit comprises a capacitance-to-frequency converter (213) local to plurality of cMUT elements, and a loop filter (410) and frequency reference source (412) remote from the plurality of cMUT elements.

6. The device of any one of the preceding claims, wherein the controller (200) is part of an ASIC (202) monolithically integrated with the cMUT device (204).

7. The ultrasound device of any one of claims 1 to 2, wherein the pressure-dependent electrical characteristic comprises a resonance frequency.

8. The device of claim 1, wherein a first sub-array (600) of the cMUT device is used for ultrasound transmission and reception and a second sub-array (610) of the cMUT device is used for measuring pressure values of body liquid.

9. The device of any one of claims 1 to 8, wherein the device is configured for blood pressure measurement, ultrasound imaging of body anatomy and/or blood flow velocity measurement.

10. The device of any of the preceding claims, wherein the imaging mode comprises a sequence of frames (300), and the pressure measurement comprises measurements performed between frames (300) of the imaging mode.

11. The device of any one of claims 1 to 10, wherein the controller is operable in a Doppler flow velocity measurement mode, in which the transmit circuit delivers pulses to the one or more elements of the cMUT device and the ultrasound receive circuit receives echo signals.

12. The device of any one of claims 1 to 11, wherein the device is one of an introducer sheath, a catheter and a guidewire.

13. A system comprising:
a device of any one of claims 1 to 12; and
a display system for displaying as a combined image two or more of:
ultrasound images;
flow velocity information;
pressure information.

14. The system of claim 13, further comprising a tracking system for tracking the location of the device based on ultrasound emitted or received by the cMUT device, and wherein the display is further configured for displaying location information of the device.

15. A method of performing ultrasound imaging and pressure sensing using a device comprising a shaft and one or more cMUT elements at a distal portion (10) of the shaft, wherein the method comprises:
using an integrated circuit controller to control an ultrasound transmit circuit and an ultrasound receive circuit to perform ultrasound imaging and/or body liquid flow velocity measurement; and
using the integrated circuit controller for measuring a pressure-dependent electrical characteristic of the one or more of the cMUT element and deriving body liquid pressure measurement values.
